Europäisches Patentamt

European Patent Office

Office européen des brevets

(11) Veröffentlichungsnummer: **0 262 638**
**A2**

(12) **EUROPÄISCHE PATENTANMELDUNG**

(21) Anmeldenummer: 87114144.6

(22) Anmeldetag: 28.09.87

(51) Int. Cl.4: **A61F 13/00** , A61F 13/06 , A61F 13/10

(30) Priorität: 26.09.86 DE 8625798 U

(43) Veröffentlichungstag der Anmeldung:
06.04.88 Patentblatt 88/14

(84) Benannte Vertragsstaaten:
AT BE CH DE ES FR GB GR IT LI LU NL SE

(71) Anmelder: **Firma Heinz Schiebler**
**Gummistrumpffabrik**
**Marienallee 74**
**D-2390 Flensburg(DE)**

(72) Erfinder: **Schiebler,Heinz**
**Marienalle 74**
**D-2390 Flensburg(DE)**

(74) Vertreter: **Richter, Werdermann & Gerbaulet**
**Neuer Wall 10**
**D-2000 Hamburg 36(DE)**

(54) **Kompressionseinlage für eine Bandage.**

(57) Kompressionseinlage für eine Bandage Die Kompressionseinlage (100) für eine Bandage aus einem elastischen Gewebe, Gewirke oder Gestricke, insbesondere in Form eines Schlauches , zur Kompression von Knie-, Spring-, Ellenbogen-und/oder Handgelenken, die im angelegten Zustand der Bandage die Knochenvorsprünge des Gelenkes umgibt und die die benachbarten Gelenkweichteile beaufschlagt, besteht aus einem Formkörper (10) aus einem elastischen, kompressiblen, durch Druck verformbaren und ohne Sprungelastizität in seine Form sich rückbildenden Silikonkautschuk vom Typ eines nach dem Verfahren der Polyaddition vulkanisierenden Kaltkautschuks, der eine hohe Flexibilität bei einer ShoreFestigkeit unter 4° der Skala A aufweist, so daß zwei gleichzeitig wirkende therapeutische Effekte, nämlich durch Wechselbelastung der druckbeaufschlagten Gelenkweichteile und nach Beendigung der Kompressionsphase durch den verdichteten Massenbereich der Kompressionseinlage eine Massagewirkung, und zwar auch in den druckunbeaufschlagten Bereichen der Gelenkweichteile, erhalten werden.

FIG.1

## Kompressionseinlage für eine Bandage

Die Erfindung betrifft eine Kompressionseinlage für eine Bandage aus einem elastischen Gewebe, Gewirke oder Gestricke, insbesondere in Form eines Schlauches, zur Kompression von Knie-, Spring-, Ellenbogen-und/oder Handgelenken, wobei die Kompressionseinlage im angelegten Zustand der Bandage die Knochenvorsprünge des Gelenkes umgibt und die benachbarten Gelenkweichteile beaufschlagt.

Nach "Orthopädie-Technik", Dezember 1961, Heft 9, Seite 253, ist es bekannt, zur Vermeidung von in Amputationsstümpfen äuftretenden Zirkulationsstörungen und von venösen Stauungen im Innenraum von Oberschenkelkontaktschäften Stumpfkissen anzuordnen oder die Schäfte mit einem federnden Schaftboden zu versehen, auf dem sich der Amputationsstumpf abstützt. Diese Stumpfkissen sind elastisch ausgebildet und bestehen aus Schaumstoffen, wie z.B aus dem unter dem Handelsnamen bekannten Moltopren, bei dem es sich um einen Polyurethan-Schaumstoff handelt. Durch ein derartiges elastisches Stumpfkissen wird ein inniger Kontakt des Stumpfspitzengebietes mit dem Schaftboden erzielt und durch das Einsinken des Stumpfes bei Belastung das Stumpfspitzengebiet leicht komprimiert. Während der Schwungphase der Prothese verringert sich dieser Kontakt wieder und durch dieses Wechselgeschehen wird eine echte Massage des Stumpfendes erreicht, was als wesentliche Unterstützung der Muskelpumpe zur Förderung der Zirkulation in diesem Stumpfgebiet gewertet werden darf. Aufgrund der hohen Elstizität und insbesondere des geringen Rückstellvermögens das Polyurethan-Schaumstoffe besitzen, wird zwar eine Massagewirkung erreicht, die sich jedoch mehr oder weniger auf den Oberflächenbereich des Stumpf spitzengebietes erstreckt und mit kiener Tiefenwirkung verbunden ist. Hinzu kommt, daß die Elastizität insbesondere von weichen Polyurethan-Schaumstoffen bei häufiger Druckbelastung nachläßt, so daß die gewünschte Massage nicht immer voll zur Wirkung kommt.

Durch die EP-A-0027172 ist eine Bandage aus elastischem Bandagenstoff, insbesondere in Schlauchform, für die Abstützung bzw. Kompression von Knie-, Sprung-, Elllenbogen-und/oder Handgelenken mit wenigstens einer im angelegten Zustand die Knochenvorsprünge des Gelenkes umgebenden, die benachbarten Gelenkweichteile beaufschlagenden Kompressionseinlage bekannt, die aus elastischem, jedoch inkompressiblem Silikonkautschuk oder einem Material mit gleichen Elastizitäts-und Kompressionseigenschaften besteht. Durch die Verwendung eines elastischen, jedoch inkompressiblen Silikonkautschuks als Material für die Kompressionseinlage wird bei angelegter Bandage eine sich nur auf die Oberfläche der die Gelenkweichteile beaufschlagenden Kompressionseinlage erstreckende Massagewirkung ohne Tiefenwirkung erhalten, da ein Silikonkautschuk, der elastische und imkompressible Eigenschaften aufweisen soll, eine geringe Rückprallelastizität aufweist, so daß die für eine Massagewirkung erforderliche Materialverschiebung bei Druckbeaufschlagung nicht eintritt und eine Massage mit einer Tiefenwirkung nicht erreichbar ist. Hinzu kommt noch, daß es für eine gute Durchblutung der Gelenkweichteile erforderlich ist, wenn eine ausreichende Wechselbelastung erzeugt wird, aufgrund der das Blut aus den unter der Kompressionseinlage befindlichen Gelenkweichteilen heräusgedrückt und bei Entlastung wieder zurückströmt. Mit einem elastischen, inkompressiblen Silikonkautschuk ist aber eine derartige Wechselbelastung nicht erreichbar, da ausgeübte lokale Druckkräfte auf die der Druckkomponente zugeordnete Abstützfläche nicht erzeugbar sind. Das ist darauf zurückzuführen, daß der bei dieser Bandage als Kompressionseinlage eingesetzte Silikonkautschuk bei Druckeinwirkung nicht komprimiert wird, sondern es tritt lediglich eine Massenverschiebung in dem Sinne ein, daß die von den druckbeanspruchten Bereichen verdrängten Massenanteile seitlich auswandern, und zwar in benachbarte Bereiche, die nicht druckbeansprucht sind, so daß in diesen benachbarten Bereichen eine Massenvermehrung eintritt, die in Verbindung mit der angelegten Bandage auf die entsprechenden Bereiche des Gewebes eine Massagewirkung ausüben. Eine voranstehend erwähnte Wechselbelastung wird aber nicht erreicht; zwar eine gute Oberflächenmassage, aber keine Massage mit Tiefenwirkung und somit keine Durchblutungsverbesserung.

Ferner ist durch die US-A-3,458,867 eine Bandage mit einer Kompressionseinlage bekannt, die ringförmig ausgebildet ist und aus einem Polyurethan-Schaumstoff besteht. Bei angelegter Bandage wird eine Massagewirkung erreicht.

Ein Fußgelenk-und Knöchelschutz mit einem im Bereich eines vorstehenden Teiles eines Fußknöchels anbringbaren Polsterwulst aus hochelastischem Schaumstoff, der so stark bemessen ist, daß seine äußere Wulstfläche die Kuppe des Knöchels noch überragt, ist nach der DE-A-1015364 bekannt. Der Polsterwulst umschließt dabei U-förmig den auf der Außenseite des Fußgelenkes hervortretenden Teil des Knöchels unter Druck von unten und von beiden Seiten, wodurch eine Stützung und ein Schutz des Knöchelgelenkes und darüber hinaus auch eine Förderung der Durchblutung des gesamten Fußgelenkes durch Massage beim Bewegen des Fußes erreicht werden soll.

Ein Druckkissen für Kompressionsverbände ist durch die AT-A-181686 bekannt Hier besteht das Druckkissen aus einem Gummiformkörper aus Schaumgummi, wodurch eine Unterstützung der Muskelwirkung bei der Pumptätigkeit, die auf das venöse System ausgeübt wird, erfolgt; er begegnet einer venösen Stauung und beseitigt die daraus enstehenden Ödeme, so daß auf diese Weise das Beingeschwür oder Ödem durch Entstauung heilt.

Die bei den bekannten Bandagen verwendeten Kompressionseinlagen bestehen entweder aus Schaumkunststoffen oder aus einem elastischen, inkompressiblen Silikonkautschuk, wobei im ersteren Fall im wesentlichen nur eine gute Durchblutung oder eine Durchblutungsförderung erreicht wird, während im zweiten Fall durch Massenverschiebung eine Massagewirkung ohne Tiefenwirkung erzielt wird .

Die Erfindung löst die Aufgabe, eine Kompressionseinlage für Bandagen mit einer guten Wärmeleitfähigkeit und mit gegenüber den bekannten Kompressionseinlagen verbesserter Wirkung zu schaffen, die derart elastisch und flexibel ist, daß sie sich dem Muskelspiel und dem Bewegungsspiel der abzudeckenden Gelenkweichteile anpaßt, so daß in Verbindung mit dem Zusammenspiel von Muskelbewegungen und Gelenkweichteilbewegungen sowie der Materialeigenschaften eine tiefwirkende Massage bei gleichzeitig verbesserter Durchblutungsförderung durch Schaffung einer stetig wechselnden und wandernden Kompressionszone sowohl in der Tiefe als auch auf der Oberfläche der Gelenkweichteile erreicht wird.

Diese Aufgabe wird durch die im Patentanspruch 1 gekennzeichneten Merkmale gelöst.

Mit einer derart ausgebildeten Einlage für Bandagen wird aufgrund der Ausgestaltung der Einlage und der Werkstoffauswahl für deren Herstellung eine hohe Kompressions-und damit verbunden eine hohe Massagewirkung erreicht, die sich nicht nur auf die Oberfläche der abgedeckten Gelenkweichteile erstreckt, sondern mit einer hohen Tiefenwirkung verbunden ist. Außerdem erstreckt sich die Massagewirkung nicht ausschließlich auf die den Knochenvorsprüngen des Gelenkes benachbarten Gelenkweichteile, sondern durch die hohe Massagewirkung und der bei der Druckausübung erfolgten Materialkompression und anschließenden Massenverschiebung in der Kompressionseinlage und der gleichzeitigen Ausbildung auf der Oberfläche der Gelenkweichteile wandernder Kompressionszone von wechselnder Stärke erstreckt sich die Massagewirkung auch auf die Knochenvorsprünge. Hinzu kommt, daß der verwendete Werkstoff eine gute Wärmeleitfähigkeit aufweist, so daß es nach dem Anlegen der Bandage nicht zu Wärmestaus kommt, da eine gleichmäßige Ableitung der Körperwärme im Bereich der angelegten Bandage,und zwar über den gesamten Bereich des Formkörpers der Kompressionseinlage, erfolgt, jedoch auf der anderen Seite bei der Verwendung entsprechenden Bandagenmaterials die Körperwärme im Bereich der angelegten Bandage mit der Kompressionseinlage gehalten wird, so daß in Verbindung mit der erfolgten Massage die Durchblutung verbessert und gefördert wird. Durch die ständig auftretenden Relativverschiebungen zwischen Gelenk und Gelenkweichteilen auf der einen Seite und der Kompressionseinlage auf der anderen Seite in Verbindung mit dem durch Gelenkbewegung ausgeübten Druck auf die Kompressionseinlage werden gute Massagewirkungen erzielt.

Durch die Verwendung eines elastischen und kompressiblen Materials, wie einem diese Eigenschaften aufweisenden Silikonkautschuk, als Kompressionseinlage von Bandagen wird bei einer Druckbelastung neben einer Tiefenwirkung mit einer Durchblutungsförderung auch eine Massagewirkung erreicht. Wird auf die Kompressionseinlage ein Druck ausgeübt, dann erfolgt zunächst eine Materialkompression aufgrund der in Richtung des ausgeübten Druckes auftretenden Druckkräfte und eine Druckbeaufschlagung der Gelenkweichteile. Bei Druckentlastung tritt eine Pumpwirkung auf, die zu einer Verbesserung der Durchblutung beiträgt. Ist das Material bei Druckeinwirkung komprimiert worden und hat es die Endphase der Kompression erreicht, dann führt eine weitere Druckeinwirkung zu einer Massenverschiebung in die seitlichen Bereiche der Kompressionseinlage mit der Folge, daß eine Massagewirkung erzielt wird. Durch die Verwendung eines insbesondere weich-elastischen und kompressiblen Materials werden mit einem einzigen Werkstoff zwei therapeutische Wirkungskomponenten erzeugt und erhalten, die hintereinander folgend und zum Teil überlagernd zur Wirkung kommen, nämlich einmal eine Durchblutungsförderung und zum anderen eine Massagewirkung, so daß ein optimaler therapeutischer Effekt erreicht wird.

Überraschend hat es sich gezeigt, daß eine Kompressionseinlage aus einem Silikonkautschuk vom Typ eines nach dem Verfahren der Polyaddition vulkanisierenden Kaltkautschuks für die Erzielung einer hohen Kompressionswirkung sich besonders vorteilhaft eignet, und zwar insbesondere dann, wenn dieser Silikonkautschuk eine hohe Flexibilität bei einer Shore-Festigkeit unter 4° der Skala A aufweist.

Durch Druckeinwirkung und Druckaufhebung erfolgt bei einer derart ausgebildeten Kompressionseinlage eine ständige Wechselwirkung in der Verschiebung der Masseteilchen, wobei gerade diese Wechselwirkung zu einer Massage mit Tiefenwirkung beiträgt, da der verwendete Werkstoff für die Herstellung der Kompressionseinlage aufgrund seiner Eigenschaften dem Muskelspiel und der Bewegung der Gelenkweichteile zwar eine ausreichende Festigkeit entgegensetzt, jedoch in Verbindung mit dem Zusammenspiel von Muskel-und Gelenkweichteilbewegung einerseits und den Materialeigenschaften des verwendeten Werkstoffes andererseits eine Massagewirkung mit gleichbleibender Stärke erzielt wird. Weitere vorteilhafte Ausgestaltungen der Erfindung sind in den Unteransprüchen gekennzeichnet. Hierbei ist besonders vorteilhaft die Ausgestaltung des Formkörpers auf der die Gelenkweichteile beaufschlagenden Seite mit einer Oberflächenprofilierung z.B. in Form eines Wellenprofils oder in Form einer Anzahl von borstenähnlich angeordneten Noppen, deren freie Enden als besonders großflächige Auflageflächen ausgebildet sind, so daß aufgrund eines ständig wechselnden Druckes die Noppen an den Bewegungen teilnehmen und aufgrund der großen Auflageflächen auch noch zusätzlich punktförmige Massagewirkungen auslösen.

Weitere Einzelheiten, Merkmale und Vorteile der Erfindung werden anhand von Ausführungsbeispielen in der Zeichnung näher erläutert. Es zeigt

Fig. 1 in einer Ansicht von oben eine Kompressionseinlage,

Fig. 2 einen senkrechten Schnitt gemäß Linie II-II in Fig. 1

Fig. 3 in einem senkrechten Schnitt eine weitere Ausführungsform einer Kompressionseinlage mit konisch sich zum umlaufenden Rand verjüngenden Wandflächen,

Fig. 4 in einem senkrechten Schnitt eine weitere Ausführungsform einer Kompressionseinlage mit sich zum umlaufenden Rand konisch erweiternden Wandflächen,

Fig. 5 eine Kompressionseinlage mit einer Oberflächenprofilierung an der den Gelenkweichteilen zugekehrten Wandfläche in einer Ansicht auf die Oberflächenprofilierung,

Fig. 6 einen senkrechten Schnitt gemäß Linie VI-VI in Fig. 5,

Fig. 7 eine weitere Ausführungsform einer Kompressionseinlage mit einer andersartig ausgestalteten Oberflächenprofilierung in einer Ansicht auf die die Oberflächenprofilierung tragende Wandfläche,

Fig. 8 in einem senkrechten Schnitt eine Kompressionseinlage mit an der den Gelenkweichteilen zugekehrten Wandfläche angeformten Noppen,

Fig. 9 in einem vergrößerten senkrechten Schnitt eine Noppe mit verbreiterter Auflagefläche,

Fig.10 in einem vergrößerten senkrechten Schnitt eine Noppe mit kreisbogenförmig ausgebildeter Auflagefläche,

Fig.11 in einer Ansicht von oben eine schlauchförmige Bandage mit an dieser angeordneten Kompressionseinlage, und

Fig.12 in einem vergrößerten senkrechten Schnitt eine weitere Ausführungsform einer Kompressionseinlage aus einem elastischen und kompressiblen Sandwichmaterial aus einem Silikonkautschuk und einem Silikonschaumstoff.

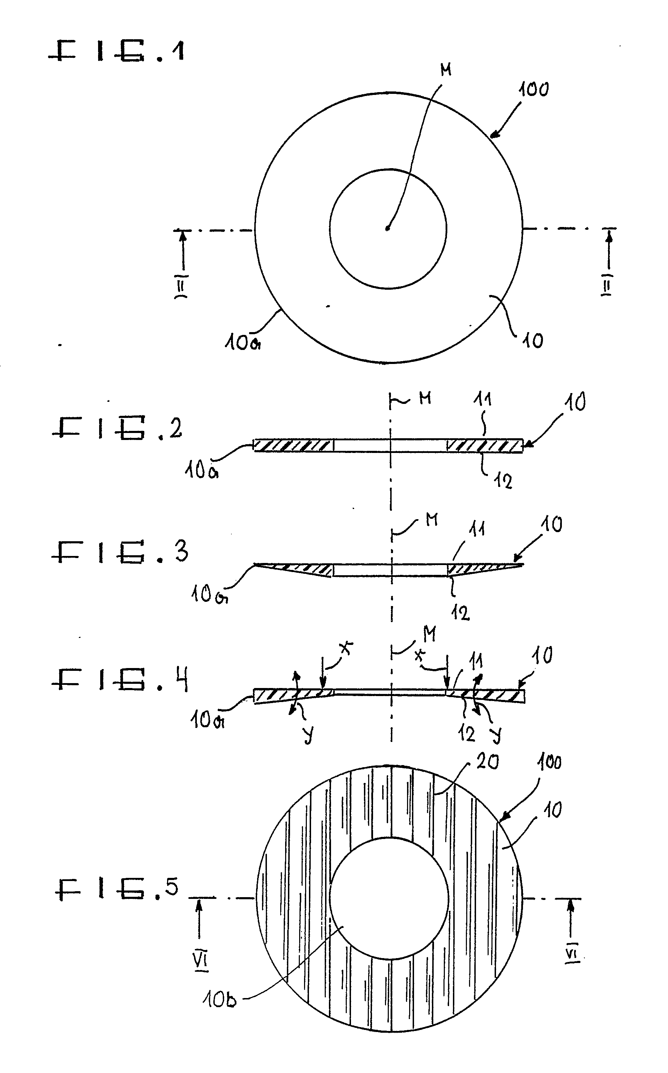

Die mit 100 in Fig. 1 bezeichnete Kompressionseinlage findet Verwendung in einer vorzugsweise schlauchförmig ausgebildeten Bandage aus einem elastischen Bandagenstoff, z.B. einem elastischen Gewebe, Gewirke oder Gestricke (Fig.11). Eine derartige Bandage 150 ist aufziehbar auf ein Knie-, Sprung-, Ellenbogen-und/oder Handgelenk, wobei die Kompressionseinlage 100 im angelegten Zustand der Bandage 150 die Knochenvorsprünge des Gelenks umgibt und dabei die benachbarten Gelenkweichteile beaufschlagt Für die Befestigung der Kompressionseinlage 100 an der Bandage 150 ist diese im Befestigungsbereich doppellagig ausgebildet, wobei dann in dem durch diese Doppellagigkeit ausgebildeten Zwischenraum die Kompressionseinlage angeordnet ist, die, wie in Fig. 1 dargestellt, ringförmig ausgebildet ist, jedoch auch eine Scheibenform aufweisen kann, wobei es dann jedoch von Vorteil ist , wenn der das Gelenk beaufschlagende Bereich einer derartigen scheibenförmigen Kompressionseinlage in seiner Stärke gegenüber der Stärke desjenigen Bereichs der Kompressionseinlage geringer bemessen ist, der die Gelenkweichteile beaufschlagt. Der im Gelenk liegende mittige Bereich der Kompressionseinlage kann in Form eines dünnen Häutchens auch ausgebildet sein. Die Stärke der Kompressionseinlage sollte bis etwa 5 mm betragen, wobei jedoch jeweils in Abhängigkeit von der vorgesehenen Anwendung und zu erzielenden Kompressionswirkung auch größere Dicken bzw. Stärken zur Anwendung gelangen können.

Entsprechend der in Fig. 1 gezeigten Ausführungsform besteht die Kompressionseinlage 100 aus einem ringförmigen Formkörper 10, der jedoch auch eine ovale, d.h. ellipsenförmige, Formgebung aufweisen kann. Die Oberfläche des Formkörpers 10 ist mit 11 bezeichnet, seine die Gelenkweichteile bei angelegter Bandage beaufschlagende untere Seite mit 12. Die Mitte bzw. die durch die Mitte gelegte Senkrechte ist bei M angedeutet. Der seitlich umlaufende Rand ist mit 10a bezeichnet.

Der Formkörper 10 der Kompressionseinlage 100 besteht aus einem kompressiblen, durch Druck verformbaren und ohne Sprungelastizität in seine Form sich rückbildenden Silikonkautschuk vom Typ eines nach dem Verfahren der Polyaddition vulkanisierenden Kaltkautschuks, der neben einer hohen Flexibilität bei einer Shore-Festigkeit unter 4° der Skala A eine gute Wärmeleitfähigkeit aufweist. Auch über 4° der Skala A liegende Shore-Festigkeiten können eingesetzt werden.

Der hier verwendete Silikonkautschuk ist ein sogenannter Kaltkautschuk, der nach dem Verfahren der Polyaddition vulkanisiert wird, wobei als auslösender Katalysator Platin verwendet wird. Es werden bei diesem Verfahren 5 bis 8 gr/100 kg gelöstes Platin diesem Material in der B-Komponente zugeführt, wodurch die Polyaddition eingeleitet wird. Dieser Vulkanisationsprozeß kann darüber hinaus über eine Temperaturzuführung in seinem Ablauf beschleunigt werden. Die besonderen Eigenschaften dieses Materials sind gekennzeichnet durch eine hohe Flexibilität (Shore-Festigkeit unter 4° der Skala A).

Derartige Silikonkautschuke fallen in die Reihe der RTV-Silikonkautschuke (vgl. Veröffentlichung Wacker-Silikone, elastische Formen aus RTV-2 Silikonkautschuk für Industrie und Handwerk, Nr. 3274.805). Wesentlich ist bei diesen Typen, daß die Härtung bei Raumtemperatur erfolgt; die Bezeichnung "RTV" steht dabei für Raum-Temperatur-Vulkanisation. Der weitere Vorteil, der sich bei der Anwendung dieser Silikonkautschuke ergibt, liegt darin, daß diese Silikonkautschuke durch Druck zwar verformbar, aber ohne Sprungelastizität in ihrer Form sich zurückbilden können, d.h. diese Silikonkautschuke weisen eine Stoßelastizität bzw. Rückprallelastizität auf, die vorzugsweise oberhalb von 40% liegt. Überraschenderweise hat es sich gezeigt, daß der zur Herstellung der Kompressionseinlage verwendete Silikonkautschuk die Eigenschaft besitzt, bei angelegter Bandage mit einer derartigen Kompressionseinlage aufgrund der gleitenden Bewegung, ausgelöst durch Masseverschiebung, bei Druckanwendung oder bei Bewegungsabläufen im Auflagebereich der Kompressionseinlage massierend zu wirken, ohne dabei einen Rolleffekt zu erzeugen, der nämlich den Nachteil hat, nicht so intensiv eine Massage zu bewirken, wie dies mit dem erfindungsgemäß angewandten Silikonkautschuk erfolgt.

Der Formkörper 10 der Kompressionseinlage 100 kann so ausgebildet sein, daß der Formkörper von gleichbleibender Stärke ist, wie dies aus Fig. 2 ersichtlich ist.

Die Massagewirkung kann aber noch zusätzlich durch eine ganz spezielle Profilgebung des Formkörpers 10 unterstützt werden. Derartige Formkörperausgestaltungen sind in den Fig. 3 bis 10 dargestellt.

So besteht die Möglichkeit, daß der Formkörper 10 eine Ausgestaltung aufweist, bei der die beiden Formkörperwandflächen, und zwar von der Mitte M ausgehend, konisch sich zu dem umlaufenden Rand 10a verjüngen. Bei der in Fig. 3 gezeigten Ausführungsform ist die Oberfläche 11 in einem rechten Winkel zu der durch die Mitte gelegten senkrechten Ebene M verlaufend, während die Wandfläche der Unterseite 12 zum umlaufenden Rand 10a konisch verjüngt verlaufend ist. Nach einer anderen Ausgestaltung weist der Formkörper 10 eine Oberfläche 11 auf, die im rechten Winkel zur mittigen Senkrechten M verläuft, während die Unterseite 12 von der Mitte ausgehend sich zum umlaufenden Rand 10a konisch verbreitert (Fig.4). Bei einer Druckeinwirkung in Pfeilrichtung X erfolgen in den Randbereichen Abschnittsverschiebungen in Richtung der Pfeile Y in der Weise, daß in den Druckangriffspunkten benachbart zur Mitte des Formkörpers der druckbeaufschlagte Formkörperabschnitte nach unten, also auf die Gelenkweichteile, gedrückt wird, während der umlaufende Rand bzw. die Endabschnitte wiederkehrende Bewegungen in Pfeilrichtung Y ausführen, so daß durch diese Randbewegung dann noch eine zusätzliche Massagewirkung erzielt wird, wobei diese Randbewegung noch durch die Masseverschiebung unterstützt wird, die bei einer Druckanwendung auf den Formkörper erreicht wird, während das vorhandene Rückstellvermögen des Formkörpers eine Rückführung der verformten Formkörperbereiche in ihre Ausgangslage bewirkt.

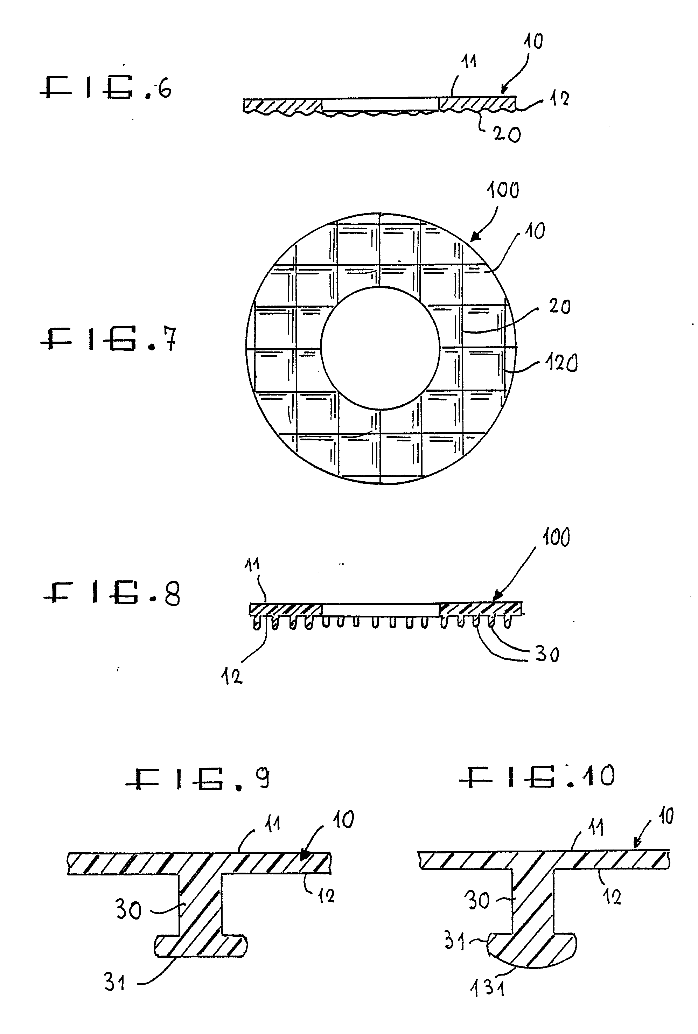

Die Massagewirkung wird auch noch zusätzlich unterstützt durch eine auf der Unterseite 12 des Formkörpers ausgebildete Oberflächenprofilierung z.B. in Form eines Wellenprofils (Fig. 5 und 6). Nach Fig. 7 besteht die Oberflächenprofilierung aus zwei sich kreuzenden Wellenprofilen 20,120.

Die Oberflächenprofilierung an der Unterseite 12 des Formkörpers 10 kann auch vermittels aus dem Material des Formkörpers ausgebildeter, senkrecht stehender Noppen 30 erreicht werden, wobei diese Noppen nach Art der Borsten eines Bürstenkörpers angeordnet sind, jedoch aus dem gleichen Werkstoff bestehen können, aus dem auch der Formkörper hergestellt ist.

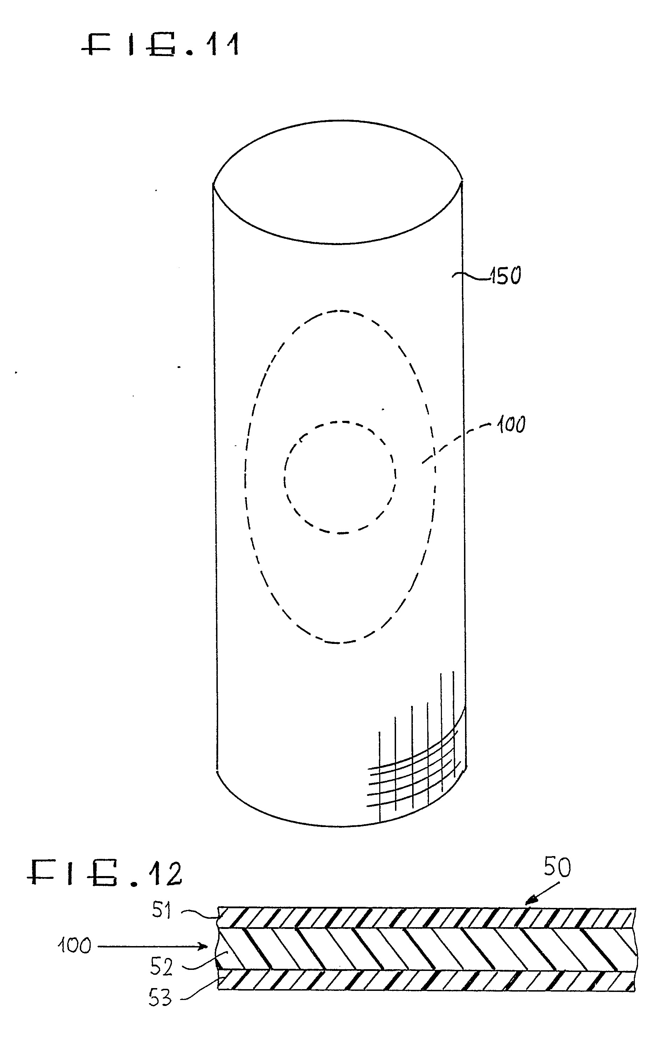

Die freien Enden dieser Noppen 30 können, wie Fig. 9 und 10 zeigen, eine ganz spezielle Ausgestaltung aufweisen. Jede Noppe 30 ist an ihrem freien Ende 31 mit einer verbreiterten Auflagefläche 31 versehen, die dadurch erhalten wird, daß an das freie Ende 31 der Noppe 30 z.B. ein scheibenförmiger Abschnitt angeformt oder mit ausgebildet ist, so daß dann bei der Einwirkung eines Druckes auf den Formkörper 10 sich dieser Druck fortsetzt in die Noppen 30 und zu einer Verformung der verbreiterten Auflagefläche 31 führt, wobei diese Verformung unterschiedlicher Art ist und von dem jeweils aufgebrachten Druck abhängt, während aufgrund des Rückstellvermögens des verwendeten Silikonkautschuks die verformten Auflageflächen 31 sich selbsttätig in ihre ursprüngliche Form zurückstellen und bei allen diesen Verformungsbewegungen noch eine zusätzliche Massagewirkung ausüben. Vorteilhafterweise ist die Auflageseite der verbreiterten Auflagefläche 31 kreisbogenförmig bzw. kugelkappenförmig ausgebildet, wie dies in Fig. 10 bei 131 angedeutet ist, so daß bei einer Druckeinwirkung auf den Formkörper 10 der Druck auf die Auflagefläche 131 der Noppen 30 übertragen wird, wodurch ein abrollartiger Effekt auf den Gelenkweichteilen erreicht wird, der die Massagewirkung der Kompressionseinlage noch unterstützt.

Besonders vorteilhaft ist, wenn nach einer weiteren Ausführungsform der Erfindung bei einer ringförmigen Kompressionseinlage 100 der von dem Ringkörper 10 ausgesparte Innenraum 10b (Fig.5) voll mit dem Material ausgefüllt ist, aus dem der Ringkörper 10 besteht. Diese Materialhaut kann filmartig, also dünn, ausgebildet sein, so daß der ausgesparte Innenraum 10b des Ringkörpers 10 voll ausgefüllt ist. Dadurch wird eine ringförmige Kompressionseinlage erhalten, deren Hauptmassagewirkung bei Einwirkung eines Druckes durch den eigentlichen Ringkörper 10 erfolgt, während die dünne innere Wandfläche des Ringkörpers 10 auf die den Knöchel bedeckenden Weichteile bei Druckausübung eine zusätzliche Massagewirkung ausübt.

Bei der in Fig. 12 gezeigten Ausführungsform besteht die Kompressionseinlage 100 aus einem Formkörper 50 aus einem Sandwichelement aus Schichten aus einem elastischen und kompressiblen Material, wie z.B. einem Silikonkautschuk, mit den vorangehend beschriebenen Eigenschaften und dem angegebenen Typ und einem Schaumkunststoff, z.B. aus einem Silikonschaum, mit einer möglichst kleinen Porengröße. In jeweiliger Abhängigkeit von der Porengröße ist die Elastizität und Kompressibilität varrierbar und den jeweiligen Erfordernissen anpaßbar. Der Formkörper 50 besteht nach Fig. 12 aus zwei Schichten 51,51a aus einem elastischen, kompressiblen Silikonkautschuk und einer Zwischenschicht 52 aus einem Silikonschaumstoff, wobei auch Schaumkunststoffe aus anderen Materialien Verwendung finden können, z.B. Polyurethanschaum u.dgl.. Der Formkörper 50 kann auch aus drei oder mehr als drei Schichten aus Silikonkautschuk und den jeweiligen Zwischenschichten aus einem Schaumkunststoff bestehen.

Der Vorteil bei der Verwendung einer Kompressionseinlage aus einem Formkörper 50 besteht darin, daß eine bessere Druckverteilung und ein besserer Druckfluß innerhalb des Formkörpers 50 erfolgt mit der Folge einer Intensivierung des therapeutischen Effektes. Ein auf den Formkörper 50 ausgeübter Druck pflanzt sich einmal in Druckeinwirkungsrichtung auf die Gelenkweichteile fort, wobei maßgeblich die hohe Kompressionswirkung der Zwischenschicht aus dem Schaumkunststoff zum Einsatz kommt. Aber gleichzeitig bei einer Druckeinwirkung erfolgt eine Massenverteilung in den Schichten aus dem Silikonkautschuk und von dort eine Druckweiter - leitung wiederum in die druckbelasteten Bereiche der Schichten aus Schaumkunststoffen, so daß auch bei einer punktuellen oder abschnittsweisen Druckeinwirkung eine Massagewirkung auch in weiter gelegenen Bereichen der Gelenkweichteile erreicht wird.

Die Schichten 51,51a des Formkörpers 50 nach Fig. 12 bestehen aus einem elastischen und kompressiblen Material, insbesondere einem Kunststoff, wie Silikonkautschuk, während die Zwischenschicht 52 aus einem Schaumkunststoff besteht. Als Schaumkunststoff können alle geeigneten Schaumkunststoffe zur Anwendung gelangen, wobei die geeigneten Verbindungen, wie Kleb-oder Schweißverbindungen, eingesetzt werden, um die Zwischenschicht 52 mit den beiden anderen Schichten 51, 51a fest miteinander zu verbinden Da die Schichten 51,51a aus einem elastischen, kompressiblen Silikonkautschuk bestehen, ist es vorteilhaft, eine Zwischenschicht 52 aus einem Silikonschaumstoff zu verwenden. Als Silikonschaumstoff können z.B. eingesetzt werden Polyäther-oder Polyesterschaumstoffe mit einem Silikonzusatz, wie z.B. Polyester mit 0,2% Polydimethylsiloxan-Öl, Polyäther mit 1% Siloxan-Polyäther-Copolymeren oder Polyäther mit 1% Siloxan-Polyäther-Copolymeren und 0,05% Silikonölpaste, ferner Silikonharzschaumstoffë. Vorteilhaft ist es, wenn die Schichten 51,51a des Formkörpers 50 gegenüber der Schicht 52 eine geringere Kompressibilität aufweisen.

Es wird somit eine Kompressionseinlage 100 für eine Bandage aus einem elastischen Gewebe, Gewirke oder Gestricke, insbesondere in Form eines Schlauches, zur Kompression von Knie-, Spring-, Ellenbogen-und/oder Handgelenken, die im angelegten Zustand der Bandage die Knochenvorsprünge des Gelenkes umgibt und die die benachbarten Gelenkweichteile beaufschlagt, erhalten, die aus einem Formkörper 10 aus einem elastischen, kompressiblen, durch Druck verformbaren und ohne Sprungelastizität in seine Form sich rückbildenden Silikonkautschuk vom Typ eines nach dem Verfahren der Polyaddition vulkanisierenden Kaltkautschuks besteht, der eine hohe Flexiblität bei einer Shore-Festigkeit vorzugsweise unter 4° der Skala A aufweist,so daß zwei gleichzeitig oder nacheinander wirkende therapeutische Effekte, nämlich durch Wechselbelastung der druckbeaufschlagten Gelenkweichteile und nach Beendigung der Kompressionsphase durch den verdichteten Massenbereich der Kompressionseinlage eine Massagewirkung , und zwar auch in den druckunbeaufschlagten Bereichen der Gelenkweichteile, erhalten werden (Fig.1).

**Ansprüche**

1. Kompressionseinlage für eine Bandage aus einem elastischen Gewebe, Gewirke oder Gestricke, insbesondere in Form eines Schlauches, zur Kompression von Knie-, Spring-, Ellenbogen- und/oder Handgelenken, wobei die Kompressionseinlage im angelegten Zustand der Bandage die Knochenvorsprünge des Gelenkes umgibt und die benachbarten Gelenkweichteile beaufschlagt, dadurch gekennzeichnet, daß die Kompressionseinlage (100) aus einem Formkörper (10) aus einem elastischen , kompressiblen, durch Druck verformbaren und ohne Sprungelastizität in seine Form sich rückbildenden Silikonkautschuk, insbesondere vom Typ eines nach dem Verfahren der Polyaddition vulkanisierenden Kaltkautschuks, besteht, der eine hohe Flexibilität bei einer Shore-Festigkeit vorzugsweise unter 4° der Skala A aufweist, oder aus einem Material mit gleichen Eigenschaften.

2. Kompressionseinlage nach Anspruch 1, dadurch gekennzeichnet, daß der Formkörper (10) auf der die Gelenkweichteile beaufschlagenden Seite mit einer Oberflächenprofilierung z.B. in Form eines Wellenprofils (20) oder zweier sich kreuzender Wellenprofile (20,120) versehen ist.

3. Kompressionseinlage nach Anspruch 1, dadurch gekennzeichnet, daß der Formkörper (10) auf der die Gelenkweichteile beaufschlagenden Seite eine Anzahl von aus dem Formkörpermaterial ausgebildeten Noppen (30) aufweist.

4. Kompressionseinlage nach Anspruch 3, dadurch gekennzeichnet, daß die freien Enden der Noppen (30) als verbreiterte, plattenförmige Auflageflächen (31) ausgebildet sind.

5. Kompressionseinlage nach Anspruch 3, dadurch gekennzeichnet, daß die freien Enden der Noppen (30) als verbreiterte, plattenförmmige Auflageflächen (31) ausgebildet sind, die auflageseitig eine kreisbogenförmige oder kugelkappenförmige Ausgestaltung (131) aufweisen.

6. Kompressionseinlage nach einem der vorangegangenen Ansprüche 1 bis 5, dadurch gekennzeichnet, daß der Formkörper (10) von seiner Mitte (M) hin zu seinem umlaufenden Rand (10) konisch sich verjüngend ausgebildet ist.

7. Kompressionseinlage nach Anspruch 6, dadurch gekennzeichnet, daß die Oberfläche (11) des Formkörpers (10) zur mittigen Senkrechten (M) in einem rechten Winkel verlaufend ist, wobei die den Gelenkweichteilen zugekehrte Unterseite (12) von der Mitte (M) des Formkörpers zu seinem umlaufenden Rand (10a) konisch sich verjüngend ausgebildet ist.

8. Kompressionseinlage nach Anspruch 6, dadurch gekennzeichnet, daß die Oberfläche (11) des Formkörpers (10) zur mittigen Senkrechten (M) in einem rechten Winkel verlaufend ist, wobei die den Gelenkweichteilen zugekehrte Unterseite (12) des Formkörpers (10) von seiner Mitte (M) ausgehend zu seinem umlaufenden Rand (10a) konisch sich erweiternd ausgebildet ist.

9. Kompressionseinlage nach einem der vorangegangenen Ansprüche 1 bis 5, dadurch gekennzeichnet, daß der Formkörper (10) von seiner Mitte (M) ausgehend zu seinem umlaufenden Rand (10) konisch sich erweiternd ausgebildet ist.

10.Kompressionseinlage nach einem der vorangegangen Ansprüche 1 bis 9, dadurch gekennzeichnet, daß die Kompressionseinlage (100) aus einem Formkörper (50) in Sandwichbauweise aus mindestens zwei Schichten (51,51a) aus einem elastischen , kompressiblen Material, insbesondere einem Silikonkautschuk und einer zwischen den Schichten (51,51a) angeordneten weiteren Schicht (52) aus einem Schaumkunststoff, wie einem Polyäther-oder Polyesterschaumstoff mit einem Silikonzusatz oder einem Schaumkunststoff auf Silikon-Basis, besteht.

11. Kompressionseinlage nach Anspruch 10, dadurch gekennzeichnet, daß die Schichten (61,51a) des Formkörpers (50) gegenüber der Schicht (52) eine geringere Kompressibilität aufweist.

12. Kompressionseinlage nach Anspruch 1 bis 11, dadurch gekennzeichnet, daß der Formkörper (50) scheiben-oder ringförmig ausgebildet ist.

13. Kompressionseinlage nach einem der vorangegangenen Ansprüche 1 bis 9 und 12, dadurch gekennzeichnet, daß der Formkörper (10) ringförmig ausgebildet ist und daß der von dem ringförmigen Formkörper (10) umschlossene Innenraum bzw. Durchbrechung(10b) mittels eines Plättchens, eines folienartigen Zuschnittes od.dgl. aus dem Formkörpermaterial verschlossen ist, wobei das Verschlußplättchen eine gegenüber der Dicke des ringförmigen Formkörpers (10) geringere Dicke aufweist und mit dem ringförmigen Formkörper einstückig ausgebildet ist.

FIG.1

M

100

II

10a

II

10

FIG.2

M    11    10

10a    12

FIG.3

M    11    10

10a    12

FIG.4

x    M    x    11    10

10a    y    12    y

20    100

10

FIG.5

VI

10b

VI

FIG.6

FIG.7

FIG.8

FIG.9

FIG.10

FIG.11

150

100

FIG.12

50

51

100

52

53